# EUROPEAN PATENT SPECIFICATION

(11) **EP 3 003 482 B1**
(45) Date of publication and mention of the grant of the patent: **05.08.2020**
(21) Application number: 13729923.6
(22) Date of filing: 06.06.2013
(51) Int. Cl.: A61N 5/10

(54) **DEVICE FOR COLLIMATING ELECTROMAGNETIC RADIATION**
VORRICHTUNG ZUR KOLLIMATION VON ELEKTROMAGNETISCHER STRAHLUNG
DISPOSITIF DE COLLIMATION DE RAYONNEMENT ÉLECTROMAGNÉTIQUE

(43) Date of publication of application: 13.04.2016
(73) Proprietor: Universität Duisburg-Essen, 45141 Essen (DE); Technische Universität Dortmund, 44227 Dortmund (DE)
(72) Inventor: FLÜHS, Dirk, 44388 Dortmund (DE); EICHMANN, Marion, 58099 Hagen (DE)
(74) Representative: Michalski Hüttermann & Partner Patentanwälte mbB
(86) International application number: PCT/EP2013/061733
(87) International publication number: WO 2014/194959

(56) References cited:
- EP-A1- 1 529 554
- WO-A1-00/09211
- WO-A1-03/008034
- WO-A1-2013/057609
- US-A1- 2006 219 956
- BAMBYNEK M ET AL: "Fluorescence 125I eye applicator", MEDICAL PHYSICS, AIP, MELVILLE, NY, US, vol. 26, no. 11, 1 November 1999 (1999-11-01), pages 2476-2481, XP012010653, ISSN: 0094-2405, DOI: 10.1118/1.598767

## Description

The present invention relates to the field of radiation oncology. Particularly, the present invention relates to a device for collimating electromagnetic radiation suitable for use in brachytherapy.

Brachytherapy, also denoted internal radiation therapy, is a therapeutic procedure that involves placing a radiation source inside or on the body, next to or inside the tissue requiring treatment. In interstitial brachytherapy, the radiation source is placed directly in the target tissue of the affected site, while contact brachytherapy involves placement of a radiation source in a space next to the target tissue. Brachytherapy is a type of radiation therapy commonly used as an effective treatment for cancer that allows the delivering of high doses of radiation to a specific area of the body, while conventional radiation therapy (external beam irradiation) projects radiation from a radiation source outside. Hence, brachytherapy allows the application of high therapeutic doses to spatially limited target volumes causing fewer radiation side effects than external beam radiation. This allows a therapy modality denoted permanent brachytherapy, which is also known as seed implantation, wherein small radioactive seeds are placed in the tumour site and left there permanently to gradually decay. Permanent brachytherapy is for example used in the treatment of prostate cancer. Further, tumours that grow inside the eye such as small or medium melanomas can be treated by a treatment modality known as plaque brachytherapy. A plaque which is a small generally metallic object containing the radiation source e.g. radioactive seeds is surgically sutured to the outside wall of the eye, i.e., the sclera. Plaque brachytherapy delivers a highly concentrated radiation dose to the tumour.

A radioactive seed such as an iodine-125 seed is a sealed radiation source that usually emits low-energy photons. Such small radioactive seeds usually are short line sources which have a length of 4.5 mm and a diameter of 0.8 mm. Thus, the dose distribution typically has a cylindrical geometry, which is inconvenient for therapy options including the peripheral region of a target volume in which a seed is implanted or the use of seeds in radioactive plaque brachytherapy of the eye. Thus, there is a demand for methods and devices reducing radiation-induced damage to the surrounding tissue.

WO 2013/057609 A1 discloses radiotherapy sources comprising seeds housed in shells. The shells are structured to generate an anisotropic radiation field e.g. by having a grooved surface.

US 2009/0156881 A1 discloses a device suitable for treating an eye that includes a housing and a plurality of fins. The plurality of fins at least partially reside within or proximate the cavity of the housing. At least a portion of the fins is configured such that radiation emitted from one or more radiation seeds positioned in the cavity of the housing is substantially directed toward a centre portion of the eye during use. However, the resulting thickness of the applicator restricts its applicability particularly regarding structures such as the eye, and the effect of collimation is not sufficient for a variety of special cases, for instance when radiosensitive structures are positioned in close proximity to the target volume.

Therefore, the object underlying the present invention was to provide a device usable in brachytherapy that reduces radiation-induced damage to the surrounding tissue.

The problem is solved by a device for collimating electromagnetic radiation, comprising: a shielding structure at least partially surrounding a radiation source which has an opening in a transmission direction (A), a plurality of lamellae of a material that absorbs electromagnetic radiation which are positioned in the opening, and collimator channels between the lamellae which extend in the transmission direction (A), wherein the lamellae have a height (H) in the range of ≥ 10 µm to ≤ 3000 µm.

The device provides a collimator for the radiation of a small radiation source, such as an iodine-125 seed, in such way that the radiation is emitted from the source in form of an exactly directed beam having a defined opening angle and a sharp boundary perpendicular to the transmission direction. The radiation in the other directions is absorbed by the device. The device provides an advantageously improved preciseness in focussing radiation towards a small target volume such as tumours on or within the eye, while the damage to the surrounding tissue caused by radiation is markedly reduced compared to standard iodine-125 applicators. The small device is usable in standard eye applicators, and particularly is suitable for brachytherapy of widespread tumours that grow on the papilla and can not be targeted, or only by causing much damage on the optic nerve using standard applicators. The device also is denoted "micro collimator". Further, also a use of the micro collimator for interstitial and contact brachytherapy of other small tumours advantageously improves the protection of surrounding tissues at risk of radiation damage or allows brachytherapy of the periphery of a target volume. Thus, it allows a conformal therapy of small target volumes with seeds.

The radiation source can be a low-energy photon emitter providing radiation in an energy range of 10 keV to 200 keV. Suitable radioactive isotopes are for example iodine-125 (¹²⁵I) and palladium-103 (¹⁰³Pd). As used herein, the term "radiation source" particularly refers to a radioactive source material that has been adapted for use in brachytherapy. Such radiation sources are commercially available in form of small radioactive implants, so called seeds. The seeds can have the form of little capsules containing an appropriate quantity of the radioactive isotope. Such iodine-125 or palladium-103 seeds usually can be rice-sized rods or cylinders of various dimensions, for example having a length of 4.5 mm and a diameter of 0.8 mm. Such iodine-125 seeds provide a suitable radiation source for applying radiation to a target volume on or within the eye or other small target volumes. The device particularly is suitable to be used with such a seed for brachytherapy.

The shielding structure has or forms an opening in a transmission direction for the radiation of a radiation source. The shielding structure at least partially surrounds the radiation source. In embodiments, the shielding structure may surround the radiation source just leaving an opening for emitting radiation. In alternative embodiments, the shielding structure may only partially surround the radiation source, particularly if a shielding in some directions is providing by other shielding elements. For example the device is used together with or in a gold plaque shell of a COMS (Collaborative Ocular Melanoma Study) eye applicator, the gold shell will provide shielding for backward radiation. In such cases the shielding structure only need surrounding the radiation source in the directions not shielded by the plaque shell.

The shielding structure may have a cylindric geometry or substantially form a rod-shaped tube, in which a radiation source can be inserted sideways. For insertion of the seeds, the shielding structure preferably is open at the sides, and can be closed by a cover. An iodine-125 seed may substantially be covered by the shielding leaving an opening or aperture along the length of the shielding structure for emission of radiation.

The opening can be formed by protruding ends of the shielding structure. These may extend for example 500 µm beyond the seeds. The opening or aperture for emission of radiation may run along the length of the shielding structure. The opening particularly may be adapted to the seeds size. The opening may have dimensions in a range of ≥ 10 mm x 3 mm to ≤ 3 mm x 0.5 mm, in the range of ≥ 7 mm x 2 mm to ≤ 4 mm x 0.8 mm, or in the range of ≥ 7 mm x 2 mm to ≤ 5 mm x 1 mm.

The device particularly is suitable to be used with an iodine-125 or palladium-103 seed. In embodiments the device may form a single component with a seed. In an embodiment of the device, a low-energy photon emitter, preferably an iodine-125 seed, is insertably housed inside the shielding structure. The cross-section diameter of such a hull formed by the shielding structure may be in a range of > 0.5 mm to ≤ 3 mm, in the range of > 0.5 mm to ≤ 2 mm, or in the range of ≥ 0.8 mm to ≤ 1.5 mm.

In an alternative embodiment, the device is housed within the housing of the radiation source such as an iodine-125 seed. The device can be integrated within a conventional seed hull with only minor altered construction. A seed usually comprises a rod-shaped radiation source enclosed in a thin housing. The shielding structure may be applied in form of a layer on the inner surface of the housing, so being positioned around the radiation source that forms the core of the seed. The shielding structure will not entirely cover the inner surface of the housing, but leave an aperture along the length of the rod-shaped housing for emitting the radiation. The radiation source need not have the form of a full rod, preferably having the form of half a rod and such proving enough space inside the seed between radiation source and housing to position the lamellae in the opening of the shielding layer. In this embodiment, the housing of the radiation source such as a low-energy photon emitter may comprise a positioning element, preferably opposed to the opening and hence the transmission direction of the radiation. The positioning element is provided for the precise positioning of the device relative to an external target structure.

Collimators have collimator channels which extend in the transmission direction and which are formed between ridges denoted lamellae. The lamellae provide a beam shaping. In an embodiment of the device, the lamellae have a height (H) in the range of ≥ 10 µm to ≤ 3000 µm, preferably in the range of ≥ 25 µm to ≤ 1500 µm, more preferably in the range of ≥ 50 µm to ≤ 1000 µm. The height of the lamellae particularly is adapted to provide beam shaping for the dose distribution emitted by radioactive seeds used in brachytherapy. Advantageously, the height of the lamellae provides a dose distribution having a sharp boundary perpendicular to the transmission direction. Used with an iodine-125 seed particularly a height of the lamellae in the range of ≥ 50 µm to ≤ 1000 µm showed to provide very sharp radiation beams. Advantageously, the improved preciseness in focussing not only markedly reduces radiation damage to the surrounding tissue, but also allows an enhancement of the radiation dose delivered to a small target volume. This allows for a therapy of yet therapy-resistant tumours.

The degree of the achieved collimation using an orthogonal design is defined by the grid ratio. As used herein, the term grid ratio is defined as the height of the lamellae to the width of the collimator channels formed between the lamellae.

The grid ratio may be chosen to provide a desired degree of collimation. Particularly, the height of the lamellae may be adapted dependent on the width of the opening. In embodiments, the grid ratio of the height of the lamellae to the width of the collimator channels is in the range of ≥ 3:1 to ≤ 6:1. Particularly referring to a collimator design using lamellae that are arranged uniformly and at regular intervals parallel to the transmission direction (A) such a grid ratio provides a marked improvement in collimation. Referring to a collimator design using lamellae in divergent arrangement, the grid ratio may be adjusted accordingly.

The lamellae form the collimator channel walls. In an embodiment of the device, the lamellae are arranged uniformly at regular intervals and form collimator channels which are aligned in parallel to one another. The collimator channels in this embodiment extent in a linear manner. In this embodiment, the transmission direction (A) runs in parallel to the direction of the lamellae. Further, in this embodiment, the transmission direction is the same for all collimator channels. This embodiment provides a sharp emission in a defined spatial direction, suitable for the irradiation of very small target volumes.

In alternative embodiments, the lamellae may be arranged in sets of divergent assembly. The transmission direction of collimator channels formed between lamellae of divergent orientation may vary for a set of channels or from channel to channel. Such sophisticated arrangement allows for a collimation of a radiation field which may be individually shaped with regard to more extended target volumes such as organic tumours, i.e. the angle of aperture (Aor) allows for adapting the radiation field to the tumour extensions.

In embodiments, an outer set of lamellae is arranged parallel to the field edge in the transmission direction (A) and perpendicular to a selected gradient, and/or an inner set of lamellae comprises a sparser number of lamellae compared to an outer set and/or is arranged radial to the transmission direction (A).

An outer set of lamellae can form a gradient on the edge of the radiation field. Hence, an outer set of lamellae may be arranged parallel to the transmission direction (A) and perpendicular to a selected gradient. A partial shielding of the radiation source with a thickness increasing from the centre of the opening to its edge selectively reduces the radiation field in different directions. Thus, the definition of the gradient at the edge may further be improved.

An inner set of lamellae may comprise a sparser number of lamellae compared to an outer set and/or may be arranged radial to the transmission direction (A). In the centre, only photons need to be eliminated that follow a direction, which severely deviates from the transmission direction, or cross the edge of the radiation field towards increasing penetration. This allows a reduction the number of lamellae towards the centre of the opening or a radial arrangement.

Further, the lamellae alternatively may be arranged in a convergent way. As the target volume of a tumour usually is bigger than the radial and normally also the longitudinal dimensions of a seed, an alignment towards a focus point provides limited advantage.

Suitable combinations of arrangements of inner and outer sets of lamellae can be adapted subject to the shape of a selected target volume. For example, an outer set of lamellae may be arranged parallel to the transmission direction and perpendicular to a selected gradient, and an inner set of lamellae may comprise a sparser number of lamellae compared to the outer set. Thus, the emitted beam can be formed in such way that it is precisely adapted to the shape of the target volume including sufficient safety margins. For example, a full therapeutic dose can be delivered to a tumour on the papilla while the dose in the adjacent radio-sensitive optic nerve is reduced to a minimum by the sharp gradient at the field edge provided by the outer lamellae. The opening angle of the field defined by the outer lamellae on the opposite side can be adapted to the tumour thickness. An iris melanoma can be irradiated by a field with an opening angle precisely fitted to its size, thus reducing the dose delivered to surrounding radio-sensitive structures, such as the ciliary body and the cornea. A retinoblastoma with medium height but small extension of the basis can be irradiated with a pencil-like field providing minimal doses given to the area surrounding the tumour basis. Furthermore, a set of outer lamellae prevents photons emitted in the direction of the metallic plaque backing from being diffusely back-scattered there and disturbing the well-defined radiation field.

The material of the lamellae is selected to absorb electromagnetic radiation, particularly in an energy range of 10 keV to 200 keV. Suitable materials are metals of high atomic number. Preferably, the material of the lamellae is formed of or is at least essentially formed of a non radioactive noble metal selected from gold, silver, platinum, palladium, tungsten, molybdenum or alloys. Preferably the lamellae are formed of a material selected from gold or platinum. These provide particularly good physiological compatibility and absorption.

Collimator channels provide radiation-transparent channels. The channels may by a space walled by lamellae. This space may be devoid of solid material. In embodiments, the collimator channels comprise a radiation-transmitting material. The collimator channels may be filled with such radiation-transmitting material. This is advantageous in increasing the stability of the collimator structure. The radiation-transmitting material refers to material that is not or only slightly absorbent. Such material may be a plastics material with a low atomic number and low density. The polymeric material may be a polymer selected from the group comprising polyimide, polymethacrylimide, polylactide, acrylonitrile butadiene styrene, polyurethane, or epoxydes. A preferred polyimide is commercially available under the tradename Kapton®. Kapton® comprises poly(4,4'-oxydiphenylene-pyromellitimide). Further, polylactide and acrylonitrile butadiene styrene (ABS) are particularly biocompatible.

The lamellae and channel structure provide the collimating of the radiation. Further, the strength of the radiation may be modulated by additional means such as a compensator structure. A compensator structure may be a thin layer of absorbing material which can be introduced in the beam path. Preferably, the compensator has by means of a varying thickness of a homogeneous material or a varying doting with an absorbing material such as gold a varying effective thickness in relation to its absorption capacity. Hence, in embodiments, a compensator structure of varying effective thickness in relation to its absorption capacity extends over a plurality of collimator channels for a modification of the radiation field. A modification of the radiation field especially is a homogenization. This is advantageous in cases for example when the distance from the radiation source to the target volume or its extension significantly differs with direction.

Referring to the material of the shielding structure, the material is selected to absorb electromagnetic radiation, particularly in an energy range of 10 keV to 200 keV. Preferably, the material of the shielding structure is formed of a noble metal selected from gold, silver, platinum, palladium, tungsten, molybdenum or alloys. Preferably the shielding structure is formed of gold or platinum. These have the advantage to provide a very good shielding from the radiation already in form of a thin layer. Particularly a gold shielding effectively blocks radiation emitted from the seeds and prevents excessive irradiation of surrounding tissues. Already a thin layer of gold will prevent other organs from being exposed to significant amounts of inadvertent radiation emitted from the radiation source. The shielding structure can have a wall thickness in the range of ≥ 20 µm to ≤ 1500 µm, in the range of ≥ 40 µm to ≤ 500 µm, or in the range of ≥ 50 µm to ≤ 200 µm. Such wall thickness will provide a good shielding and a sufficient stability of the structure.

Preferably, the lamellae and the shielding structure are formed from an identical material, particularly from gold. In an embodiment of the device, the shielding structure and the plurality of lamellae form a one-piece structure. The lamellae and the shielding structure can be connected by a frame. The frame can be provided by lateral ridges framing the opening of the shielding structure. Further, ridges that extend vertical to the extension of the lamellae can be provided. Ridges can be provided at a regular distance over the width of the lamellae. These ridges will further stabilise the structure of the device. This embodiment has the advantage that the device is a small but stable structure and is easy to handle. A radioactive seed can be inserted in the device without difficulty and reduce the radiation expose to the medical staff during implantation. After use, the radiation source may be disposed of. Advantageously, the device may be reused.

The device comprising a radioactive source is usable for being placed directly at the site of a cancerous tumour. Advantageously, as the device is biocompatible and hardly bigger than the seed itself, it can be placed in or near a tumour and left permanently in the body. Further, the small device can be placed like a seed inside a catheter or slender tube for temporary brachytherapy of tumours. Particularly, the device easily is insertable in conventional, modified or clinic-made applicators for brachytherapy, particularly ophthalmic brachytherapy. In an embodiment, the device is insertably housed inside an applicator for applying radiation to a target volume, particularly on or within the eye. Applicators for brachytherapy, for example COMS (Collaborative Ocular Melanoma Study) eye applicators for iodine-125 ophthalmic tumour treatment, are commercially available. COMS eye applicators are assembled of a gold plaque shell combined with a silicon insert in which iodine-125 seeds are put in. Advantageously, the device can be housed inside the slots of such silicon inserts adapted for iodine-125 seeds, as the device preferably does not markedly exceed the size of a seed itself.

Another aspect of the invention refers to an applicator for applying radiation to a target volume particularly on or within the eye, comprising at least one device according to the invention and at least one radiation source, preferably an iodine-125 seed. Such applicators, which also are denoted plaques, especially are used in ocular brachytherapy. Applicators may comprise at least one preferably several devices and seeds, depending on the construction of the applicator.

The device and the applicator described herein are usable in brachytherapy. Particularly, the device or the applicator are usable in interstitial or contact brachytherapy of tumours of the prostate, mamma, brain and eye. The device containing a radiation source is usable to be placed inside the body, next to or inside a tissue requiring treatment, and can be implanted in a cancerous tissue such as tumours of the prostate, mamma, or brain. Particularly an applicator comprising one or more devices containing radiation sources preferably are usable for episcleral plaque brachytherapy. An applicator can be sutured to the outside wall of the eye, i.e., the sclera, proximate an ocular tumour such as an intraocular melanoma located therein. The applicator may remain on the eye until the intraocular melanoma has received a therapeutic dosage of radiation and after that is surgically removed. Particularly, the applicator or device is usable the treatment of macula degeneration, haemangioma, and ocular melanoma such as choroidal melanoma, conjunctival melanoma, iris melanoma, and retinoblastoma.

The disclosure refers to a method of treating cancer, comprising directing radiation towards a target volume of a tumour, particularly on or within the eye, using the device according to the invention and one or more radiation sources, or an applicator comprising such a device. The device for collimating electromagnetic radiation as described herein comprises a shielding structure at least partially surrounding a radiation source which has an opening in a transmission direction (A), a plurality of lamellae of a material that absorbs electromagnetic radiation which are positioned in the opening, and collimator channels between the lamellae which extend in the transmission direction (A), wherein the lamellae have a height (H) in the range of ≥ 10 µm to ≤ 3000 µm.

It is very advantageous, that the device allows for a considerable improvement in the preciseness in radiation treatment of tumours, particularly small tumours. The device allows for an improvement in focussing radiation towards a small target volume such as tumours on or within the eye, while the damage to the surrounding tissue caused by radiation is markedly reduced compared to standard iodine-125 applicators. The device is suitable for brachytherapy of widespread tumours that grow on the papilla that are difficult to target or using standard applicators can only be treated by causing much damage. Further, also a use of the micro collimator for interstitial and contact brachytherapy of other small tumours advantageously improves the protection of surrounding tissues at risk of radiation damage or allows brachytherapy of the periphery of a target volume. Moreover, the device is usable for high dose brachytherapy treatment.

The device may be fabricated in accordance with well-established procedures that are known to a person skilled in the art. For embodiments of the device, wherein the shielding structure and the lamellae form a one-piece structure, a suitable metal device may be manufactured by using foils manufactured by conventional rolling process, by deep-drawing or metal-casting processes, electro discharge machining, punching or laser cutting processes, or powder metallurgical process. Single metal films can be photolithographically etched and laminated one over another. CAD-based manufacturing methods permit precise and flexible production of complex 3D collimator geometries, which is particularly advantageous when different sets of lamellae are to be arranged in varying spatial direction. Advantageously, the device may be produced by 3D printing. Using 3D printing, the device can be produced in a simple way with individualized structures and high accuracy.

Particularly referring to embodiments of the device, wherein the collimator channels are filled with a polymeric radiation-transmitting material, the device may be produced by stereolithography, a substantially rapid prototyping technique. Hence, another aspect of the invention refers to a method of manufacturing a device according to the invention or an applicator comprising such an device, the method comprising the step of forming the device or the applicator including the device by a technique of stereolithography, wherein the lamellae and optionally the collimator channels are structured by doping a radiation-transmitting material with different amounts of a radiation absorbing material.

Advantageously, manufacturing the device by stereolithography allows for a production of individually patient- or tumour-tailored devices. Individual geometry, grid ratio and/or lamellae arrangement can be manufactured. A preferred technology is two-photon stereolithography, which shows an improved resolution and allows the manufacture of particularly filigree and small features in the µm range.

Manufacturing the device using a polymer-based 3D printing process and realising the physical features by mixing metal powder into a polymer matrix allows for a highly individual therapy, and at the same time is an economic method, as the seeds may be re-used, as well as the metal powder may be recovered and recycled after dissolving the polymer matrix in an appropriate solvent.

The radiation-transmitting material can be a polymer selected from the group comprising polyimide, polymethacrylimide, polylactide, acrylonitrile butadiene styrene, polyurethane, or epoxydes. A preferred polyimide is commercially available under the tradename Kapton®, comprising poly(4,4'-oxydiphenylene-pyromellitimide). Further, polylactide and acrylonitrile butadiene styrene (ABS) are usable in stereolithographic processes. The radiation absorbing material preferably is a non radioactive noble metal selected from the group comprising gold, silver, platinum, palladium, tungsten, molybdenum or alloys, preferably selected from gold or platinum. Preferred working material is gold-doted polyimide or acrylonitrile butadiene styrene.

By means of stereolithography the device is constructed as a result of layerwise solidification of a structural material under the action of radiation. A 3D-CAD structure of the geometry of an individual device can be converted into volumetric data in a CAD system, the 3D volumetric model subsequently divided into cross sections in a computer, the data transferred onto a stereolithography system, and the shape of the device constructed layer by layer by curing a liquid radiation-transmitting polymer doted with suitable amounts of the radiation absorbing material.

For structuring the lamellae the radiation-transmitting material preferably will be doped with high amounts of radiation absorbing material, while for structuring the collimator channels the radiation-transmitting material preferably comprises low amounts or none of the radiation absorbing material. Particularly compensator structures of varying effective thickness in relation to absorption capacity can easily be manufactured by doting radiation-transmitting material with finely graduated amounts of radiation absorbing material. Especially when compensator structures extend over a plurality of collimator channels stereolithography processes provide a feasible method for manufacturing. Stereolithography processes allow a wide variation of radiation intensity, and radiolucency is individually adjustable. Further, stereolithography processes allow a flexible processing. Applicators need not be manufactured in full by stereolithography. For example the backing of a conventional applicator shell may be combined with an inner applicator structure comprising the collimator device produced by stereolithography.

Unless otherwise defined, the technical and scientific terms used herein have the same meaning as commonly understood by one of ordinary skill in the art to which this invention belongs.

The examples which follow serve to illustrate the invention in more detail but do not constitute a limitation thereof.

In the figures show:
- Figure 1: shows cross sectional views of embodiments of a device according to the invention. Figure 1A shows an embodiment wherein a seed is housed inside the shielding structure of the device, while Figure 1B shows an embodiment wherein the device is housed within the housing of a seed.
- Figure 2: shows a 3-dimensional view on an embodiment of a device according to the invention. Figure 2A shows a front view, while Figure 2B shows a lateral view on the device.
- Figure 3: shows cross sectional views of an embodiment of an applicator. Figure 3A shows the backing of the applicator as a separated feature, while Figure 3B shows a cross sectional view on the assembled applicator. Figure 3C shows in detail the seed slot and lamellae.
- Figure 4: shows dose distributions, i.e. isodose curves of an iodine-125 seed inserted in a device according to the invention (bold) and an iodine-125 seed without collimation.
- Figure 5: shows Monte Carlo simulations of the measured normalized dose distributions of an iodine-125 seed using different heights of lamellae.

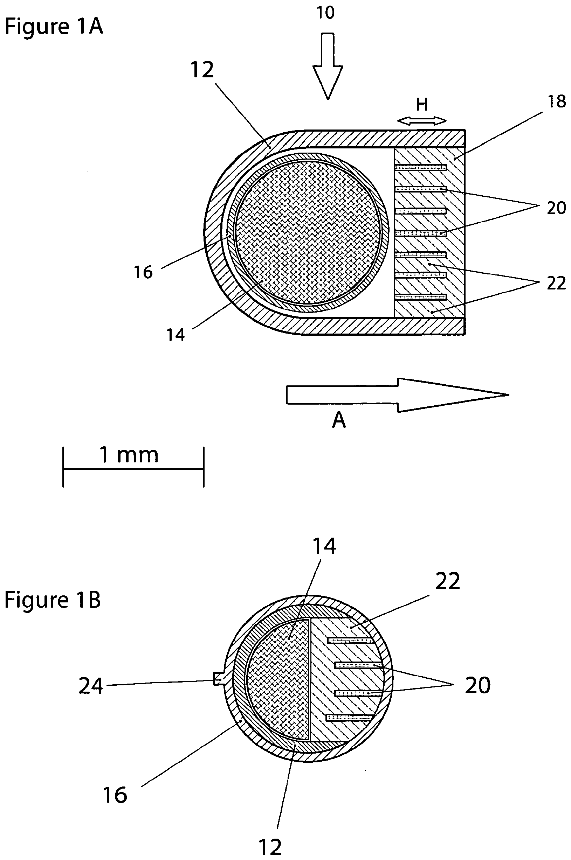

Figure 1A shows a cross sectional view of an embodiment of a device 10 for collimating electromagnetic radiation according to the invention. A shielding structure 12 around a sealed radiation source forms an opening 18 in a transmission direction A for the radiation. The sealed radiation source is an iodine-125 seed of a diameter of 0.8 mm, wherein a ceramic matrix containing iodine-125 14 is housed in a titanium housing 16. The cross-section diameter of the shielding structure is 1.2 mm. A plurality of lamellae 20 is positioned in the opening 18. The lamellae have a height H of 250 µm and are formed of gold, which is a biocompatible material of good absorption capacity for low-energy photons. The lamellae 20 are arranged uniformly at regular intervals and form collimator channels 22 between each other that are aligned in parallel to one another. The collimator channels 22 are filled with a radiation-transmitting polyimide material. The grid ratio of the height of the lamellae 20 to the width of the collimator channels 22 is 4:1.

Figure 1B shows a cross sectional view of an alternative embodiment of a device 10, wherein the device 10 is housed within a titanium housing 16, together with a ceramic matrix containing iodine-125 14. The shielding structure 12 is applied as a gold layer on the inner surface of the titanium housing 16 around the ceramic matrix containing iodine-125 14. The ceramic matrix 14 containing the radioactive material is in the form of half a rod. The lamellae 20 are positioned inside the housing 16 of the seed in an opening in the shielding layer 12. The housing 16 comprises a ridge 24 opposite the opening as a positioning element.

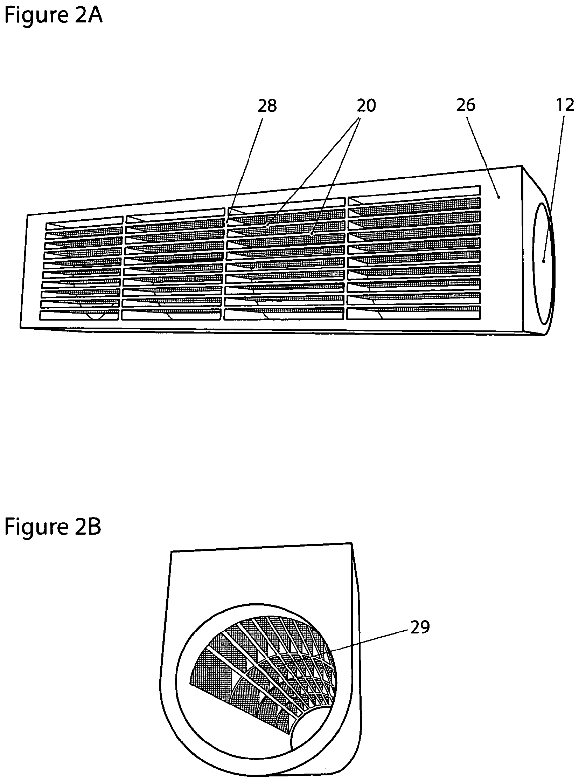

Figure 2A shows a front view of an embodiment of a device 10. In this embodiment the shielding structure 12 and the plurality of lamellae 20 form a one-piece structure. The lamellae 20 and the shielding structure 12 are connected by a frame 26. Lateral ridges frame the opening of the shielding structure to the sides. Further, ridges 28 extend vertical to the lamellae 20. These ridges further stabilise the lamellae and the one-piece device. The device has lateral openings, in which a radiation source can be inserted. The Figure 2B shows a lateral view on the one-piece device 10, showing the cavity of the seed slot 29 provided for the radiation source.

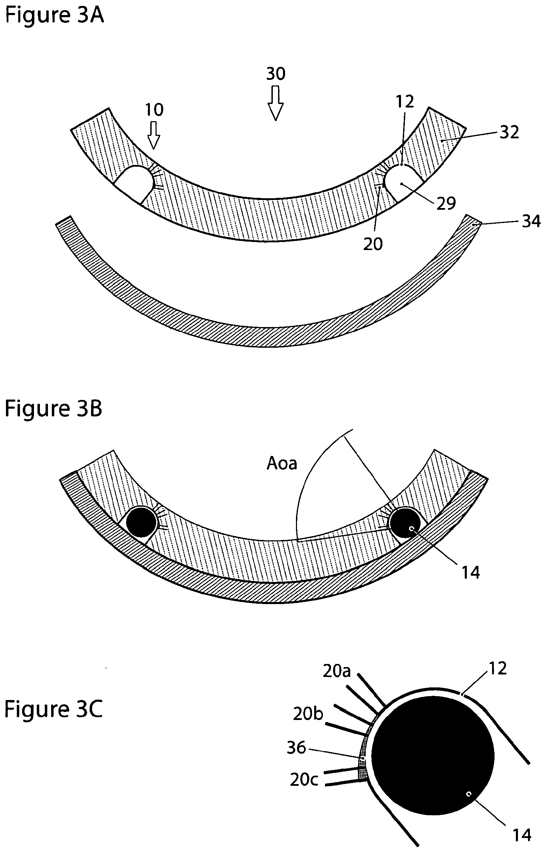

Figure 3A shows a cross sectional view of an embodiment of an applicator according to the invention. The applicator 30 has an inner layer 32, which is formed from a radiation-transmitting polyimide material by stereolithography. Integrated in the layer 32 is the device 10 with sets of lamellae 20 and shielding 12. The Figure 3A further shows a backing 34 in form of a titanium calotte. The inner concave surface of the backing 34 is covered with a gold layer for shielding backward radiation. Figure 3B shows the assembled applicator with radiation sources 14 housed inside the shielding structure 12. The angle of aperture Aoa defines the range of the directions of the radiation emission, i.e. the radiation field size. The field size is adapted to the target volume, i.e. the tumour extension between its basis and its apex.

Figure 3C shows in detail the device 10 with shielding structure 12, seed slot 29 comprising the radiation source 14 and different sets of lamellae. An outer set of lamellae 20a is arranged parallel to the upper field edge that is adapted to the tumour apex. Thus, a defined field gradient perpendicular to the radiation field allows for a sparing of structures outside of the target volume. An inner set of lamellae 20b comprises a sparser number of lamellae compared to the outer set and is arranged radial to the transmission direction A. A further set of lamellae 20c provides avoidance of backscattered radiation from the gold layer of the backing.

Figure 3C also shows a compensator structure 36 that extends over a plurality of collimator channels 22. The compensator structure 36 has varying effective thickness in relation to its absorption capacity. This allows for a homogenization of the radiation field within the target volume, since it compensates dose variations delivered to different regions of the tumour due to their changing distance to the seed. The lamellae 20 and the compensator structure 36 can be printed by stereolithography methods, wherein the lamellae are provided by highly gold doped polyimide, while the compensator structure comprises less doting.

### Example 1 - Preparation of a collimator device

A polymethylmethacrylate (PMMA) block having an overall base size of 30 mm x 30 mm x 10 mm was cut from a PMMA block (Evonik Rohm GmbH) using a bandsaw. A bore having a diameter of 0.9 mm was drilled through the length of the PMMA block, parallel to one 30 mm x 30 mm surface, using a twist drill bit. The remaining wall thickness between the bore and the block surface was 0.4 mm. An iodine-125 seed (IBt Bebig, type 125.S16) was fixed in the bore. The PMMA block was covered with a shielding layer of gold foil (Goodfellow GmbH) of 30 mm x 30 mm and a thickness of 100 µm, which formed an opening of 5 mm x 1 mm, adapted to the size and the position of the seed.

A sandwich structure was set up of 25 µm thick layers of gold foil and 100 µm thick layers of polypropylene foil (Herlitz PBS AG). The layers were fit into a slot with a size of 5 mm x 1 mm x 0.5 mm within a second PMMA block with a size of 30 mm x 30 mm x 0.5 mm. The position of the slot was adapted to the seed position, so that the gold foils formed lamellae of a height of 500 µm parallel to the direction of the emission of the seed, and the plastic foil formed collimator channels between the gold lamellae.

### Example 2 - Dosimetric measurement of a collimated and an uncollimated seed dose distribution

The collimator as described in example 1 was used for the dosimetric measurements using an iodine-125 seed (IBt Bebig, type I25.S16). The measurements were performed by a plastic scintillation dosimetry system moved by a 3D scanner in a water phantom, as described in M. Eichmann, Med. Phys. 36 (10) October 2009, p 4634-4634. This is a high-precision standard measuring tool for the dosimetry of eye applicators and seeds.

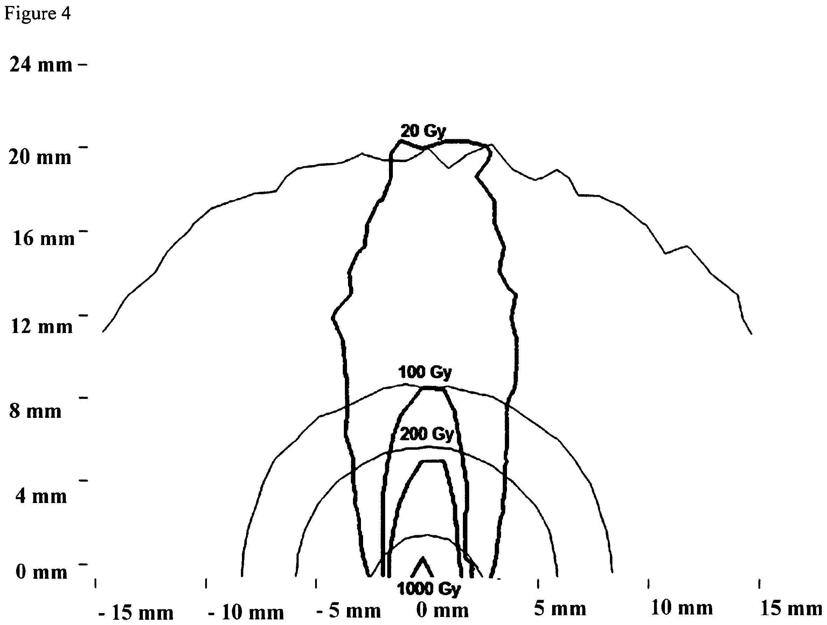

The results of the measurements are shown in Figure 4. The bold lines show the isodose curves of the radiation field of a seed collimated with the device of example 1. The radiation field laterally is strongly narrowed, compared to the radiation field of a seed without collimation. Used for brachytherapy in clinical administration a small tumour can be radiated while damage to the surrounding tissue can be reduced to the greatest possible extent. A collimation of the radiation field of an iodine-125 seed to such an extent as shown in Figure 4 was not reported hitherto.

For comparison, the thin lines show the isodose distribution emitted by an iodine-125 seed (IBt Bebig, type 125.S16) without use of the device. The dose distribution has a cylindrical geometry, showing an isotropic emission in all directions of the displayed plain. This has the consequence that not only the tumour is treated with radiotherapy but also the surrounding tissue is highly exposed to radiation.

The measuring results were in excellent accordance with results of Monte Carlo calculations simulating the effect of such a collimator on the dose distribution of an iodine-125 seed, as is shown in example 3.

### Example 3 - Numeric simulations of collimators with different lamellae heights

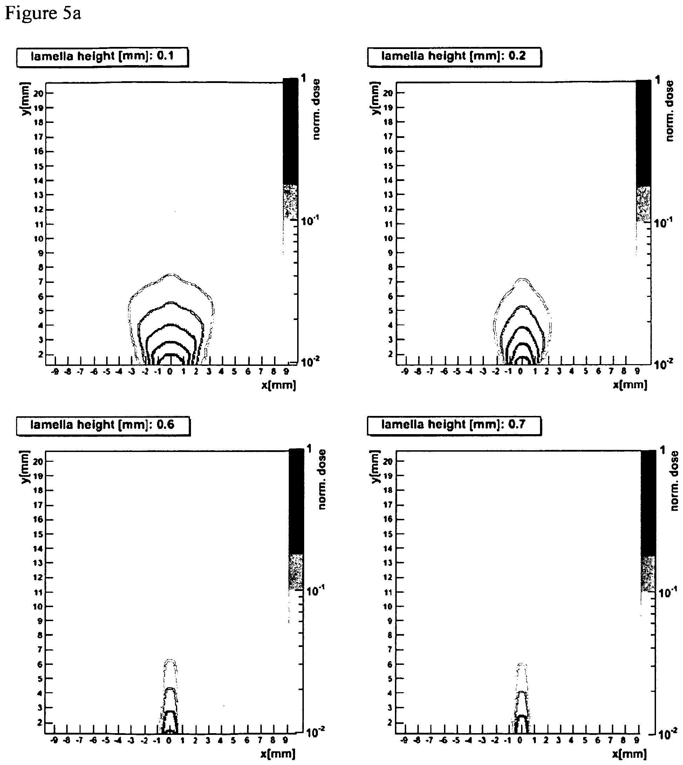

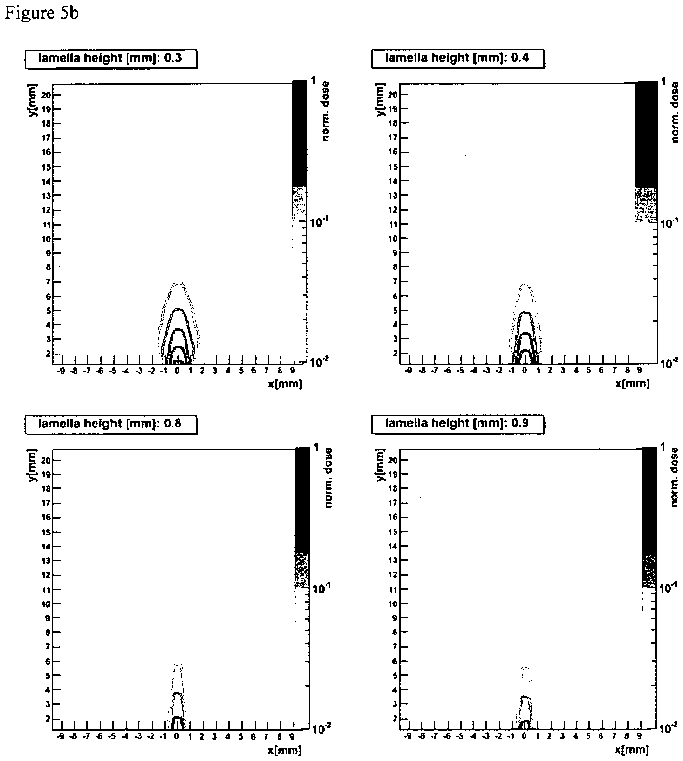

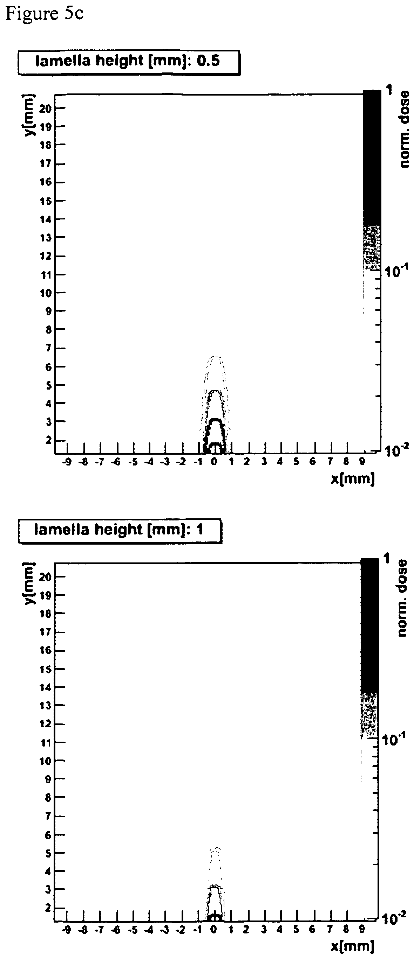

For a geometry according to Figure 1 Monte Carlo simulations of the dose distributions were performed by using the EGSnrc code, a standard Monte Carlo code for the simulation of photons in this energy range. The gold shielding (12) in these calculations was 100 µm thick, the gold lamellae (20) had a thickness of 25 µm, the collimator channels (22) were filled with Kapton® with a thickness of 100 µm. The results of these Monte Carlo simulations, isodose diagrams of the iodine-125 seed using heights of lamellae of 100 µm, 200 µm, 300 µm, 400 µm, 500 µm, 600 µm, 700 µm, 800 µm, 900 µm, and 1000 µm, are shown in Figure 5. It can be taken from the figure that with increasing height of the lamellae, the isodose distribution emitted by the seed showed a laterally increasing narrowing of the radiation field, which illustrates that a significant and increasing collimation effect was obtained. For values of 600 µm and more, an increasing saturation of the collimating effect was observed. For the dimension of the collimator used, a height of 1000 µm showed the strongest collimation, thus resulting in an almost pencil beam-like radiation field allowing irradiation techniques of highest precision.

## Claims

1. A device for collimating electromagnetic radiation, comprising:
a shielding structure (12) at least partially surrounding a radiation source (14) which has an opening (18) in a transmission direction (A), a plurality of lamellae (20) of a material that absorbs electromagnetic radiation which are positioned in the opening (18), and collimator channels (22) between the lamellae (20) which extend in the transmission direction (A),
wherein the lamellae (20) have a height (H) in the range of ≥ 10 µm to ≤ 3000 µm, wherein the grid ratio of the height of the lamellae (20) to the width of the collimator channels (22) is in the range of > 3:1 to ≤ 6:1, and wherein the radiation source is a low-energy photon emitter providing radiation in an energy range of 10 keV to 200 keV.

2. The device according to claim 1, wherein a low-energy photon emitter, preferably an iodine-125 seed, is insertably housed inside the shielding structure (12).

3. The device according to claim 1, wherein the device is housed within the housing (16) of a low-energy photon emitter, preferably an iodine-125 seed.

4. The device according to any one of the preceding claims, wherein the lamellae (20) have a height (H) in the range of ≥ 20 µm to ≤ 2000 µm, preferably in the range of ≥ 30 µm to ≤ 1500 µm, more preferably in the range of ≥ 50 µm to ≤ 1000 µm.

5. The device according to any one of the preceding claims, wherein the lamellae (20) are arranged uniformly at regular intervals and form collimator channels (22) which are aligned in parallel to one another.

6. The device according to any one of the preceding claims, wherein an outer set of lamellae (20) is arranged parallel to the field edge in the transmission direction (A) and perpendicular to a selected gradient, and/or an inner set of lamellae (20) comprises a sparser number of lamellae (20) compared to an outer set and/or is arranged radial to the transmission direction (A).

7. The device according to any one of the preceding claims, wherein the collimator channels (22) comprise a radiation-transmitting material, preferably a polymer selected from the group comprising polyimide, polymethacrylimide, polylactide, acrylonitrile butadiene styrene, polyurethane, or epoxydes.

8. The device according to any one of the preceding claims, wherein a compensator structure (36) of varying effective thickness in relation to its absorption capacity extends over a plurality of collimator channels (22) for a modification of the radiation field, especially a homogenization.

9. The device according to any one of the preceding claims, wherein the shielding structure (12) and the plurality of lamellae (20) form a one-piece structure.

10. The device according to any one of the preceding claims, wherein the device (10) is insertably housed inside an applicator for applying radiation to a target volume, particularly on or within the eye.

11. An applicator for applying radiation to a target volume particularly on or within the eye, comprising at least one device according to anyone of claims 1 to 10 and at least one radiation source (14), preferably an iodine-125 seed.

12. The device according to any one of claims 1 to 10 or the applicator according to claim 11 for use in brachytherapy, particularly interstitial or contact brachytherapy of tumours of the prostate, mamma, brain and eye, preferably for episcleral plaque brachytherapy.

13. A method of manufacturing a device according to any one of claims 1 to 10 or an applicator according to claim 11, the method comprising the step of forming the device (10) or the applicator including the device by a technique of stereolithography, wherein the lamellae (20) and optionally the collimator channels (22) are structured by doping a radiation-transmitting material with different amounts of a radiation absorbing material.

## Patentansprüche

1. Vorrichtung zur Kollimation elektromagnetischer Strahlung, umfassend:
eine Abschirmstruktur (12), die eine Strahlungsquelle (14) mindestens teilweise umgibt, welche eine Öffnung (18) in einer Durchlassrichtung (A), eine Vielzahl von Lamellen (20) eines Materials, das elektromagnetische Strahlung absorbiert, welche in der Öffnung (18) positioniert sind, und Kollimatorkanäle (22) zwischen den Lamellen (20) aufweist, welche sich in der Durchlassrichtung (A) erstrecken,
wobei die Lamellen (20) eine Höhe (H) im Bereich von ≥ 10 µm bis ≤ 3000 µm aufweisen, wobei das Gitterverhältnis der Höhe der Lamellen (20) zu der Breite der Kollimatorkanäle (22) im Bereich von ≥ 3:1 bis ≤ 6:1 liegt, und wobei die Strahlungsquelle ein niederenergetischer Photonenemitter ist, der Strahlung in einem Energiebereich von 10 keV bis 200 keV bereitstellt.

2. Vorrichtung nach Anspruch 1, wobei ein niederenergetischer Photonenemitter, vorzugsweise ein Iod-125 Seed, einführbar innerhalb der Abschirmstruktur (12) untergebracht ist.

3. Vorrichtung nach Anspruch 1, wobei die Vorrichtung innerhalb des Gehäuses (16) eines niederenergetischen Photonenemitters, vorzugsweise eines Iod-125 Seeds, untergebracht ist.

4. Vorrichtung nach einem der vorhergehenden Ansprüche, wobei die Lamellen (20) eine Höhe (H) im Bereich von ≥ 20 µm bis ≤ 2000 µm, vorzugsweise im Bereich von ≥ 30 µm bis ≤ 1500 µm, bevorzugter im Bereich von ≥ 50 µm bis ≤ 1000 µm aufweisen.

5. Vorrichtung nach einem der vorhergehenden Ansprüche, wobei die Lamellen (20) gleichförmig in regelmäßigen Intervallen angeordnet sind und Kollimatorkanäle (22) bilden, die parallel zueinander ausgerichtet sind.

6. Vorrichtung nach einem der vorhergehenden Ansprüche, wobei ein äußerer Satz von Lamellen (20) parallel zu dem Feldrand in der Durchlassrichtung (A) und senkrecht zu einem ausgewählten Gradienten angeordnet ist, und/oder ein innerer Satz von Lamellen (20), verglichen mit einem äußeren Satz, eine geringere Anzahl von Lamellen (20) aufweist und/oder radial zu der Durchlassrichtung (A) angeordnet ist.

7. Vorrichtung nach einem der vorhergehenden Ansprüche, wobei die Kollimatorkanäle (22) ein Strahlung durchlassendes Material umfassen, vorzugsweise ein Polymer ausgewählt aus der Gruppe, die Polyimid, Polymethacrylimid, Polylactid, Acrylonitril-Butadien-Styrol, Polyurethan oder Epoxyde umfasst.

8. Vorrichtung nach einem der vorhergehenden Ansprüche, wobei eine Kompensatorstruktur (36) mit, bezogen auf ihre Absorptionskapazität, variierender effektiver Dicke sich über eine Vielzahl von Kollimatorkanälen (22) erstreckt, um das Strahlungsfeld zu modifizieren, insbesondere zu homogenisieren.

9. Vorrichtung nach einem der vorhergehenden Ansprüche, wobei die Abschirmstruktur (12) und die Vielzahl der Lamellen (20) eine einteilige Struktur bilden.

10. Vorrichtung nach einem der vorhergehenden Ansprüche, wobei die Vorrichtung (10) einführbar innerhalb eines Applikators untergebracht ist, um Strahlung auf ein Zielvolumen, insbesondere auf das Auge oder innerhalb desselben, zu applizieren.

11. Applikator zum Applizieren von Strahlung auf ein Zielvolumen, insbesondere auf einem Auge oder innerhalb desselben, umfassend mindestens eine Vorrichtung nach einem der Ansprüche 1 bis 10 und mindestens eine Strahlungsquelle (14), vorzugsweise ein Iod-125 Seed.

12. Vorrichtung nach einem der Ansprüche 1 bis 10, oder Applikator nach Anspruch 11, zur Verwendung in der Brachytherapie, speziell der interstitiellen oder Kontaktbrachytherapie von Tumoren der Prostata, Mamma, des Hirns und des Auges, insbesondere zur Brachytherapie von episkleraler Plaque.

13. Verfahren zur Herstellung einer Vorrichtung nach einem der Ansprüche 1 bis 10 oder eines Applikators nach Anspruch 11, wobei das Verfahren den Schritt des Bildens der Vorrichtung (10) oder des Applikators, der die Vorrichtung einschließt, durch eine Technik der Stereolithographie umfasst, wobei die Lamellen (20) und gegebenenfalls die Kollimatorkanäle (22) durch Dotieren eines Strahlung durchlassenden Materials mit unterschiedlichen Mengen eines Strahlung absorbierenden Materials strukturiert werden.

## Revendications

1. Dispositif destiné à collimater un rayonnement électromagnétique, comprenant :
une structure de blindage (12) entourant au moins partiellement une source de rayonnement (14) qui a une ouverture (18) dans une direction de transmission (A), une pluralité de lamelles (20) d'un matériau qui absorbe le rayonnement électromagnétique qui sont positionnées dans l'ouverture (18), et des canaux de collimation (22) entre les lamelles (20) qui s'étendent dans la direction de transmission (A),
dans lequel les lamelles (20) ont une hauteur (H) dans la gamme de ≥ 10 µm à ≤ 3000 µm, dans lequel le rapport de grille entre la hauteur des lamelles (20) et la largeur des canaux de collimation (22) se situe dans la gamme de ≥ 3:1 à ≤ 6:1, et dans lequel la source de rayonnement est un émetteur de photons de faible énergie fournissant un rayonnement dans une gamme d'énergie de 10 keV à 200 keV.

2. Dispositif selon la revendication 1, dans lequel un émetteur de photons de faible énergie, de préférence un grain d'iode-125, est logé par insertion à l'intérieur de la structure de blindage (12).

3. Dispositif selon la revendication 1, le dispositif étant logé à l'intérieur du boîtier (16) d'un émetteur de photons de faible énergie, de préférence un grain d'iode-125.

4. Dispositif selon l'une quelconque des revendications précédentes, dans lequel les lamelles (20) ont une hauteur (H) dans la gamme de ≥ 20 µm à ≤ 2000 µm, de préférence dans la gamme de ≥ 30 µm à ≤ 1500 µm, mieux encore dans la gamme de ≥ 50 µm à ≤ 1000 µm.

5. Dispositif selon l'une quelconque des revendications précédentes, dans lequel les lamelles (20) sont disposées uniformément à des intervalles réguliers et forment des canaux de collimation (22) qui sont alignés parallèlement les uns aux autres.

6. Dispositif selon l'une quelconque des revendications précédentes, dans lequel un ensemble externe de lamelles (20) est disposé parallèlement au bord du champ dans la direction de transmission (A) et perpendiculairement à un gradient sélectionné, et/ou un ensemble interne de lamelles (20) comprend un nombre moins abondant de lamelles (20) par rapport à un ensemble externe et/ou est disposé radialement par rapport à la direction de transmission (A).

7. Dispositif selon l'une quelconque des revendications précédentes, dans lequel les canaux de collimation (22) comprennent un matériau transmettant le rayonnement, de préférence un polymère sélectionné dans le groupe comprenant le polyimide, le polyméthacrylimide, le polylactide, l'acrylonitrile-butadiène-styrène, le polyuréthane, et les époxydes.

8. Dispositif selon l'une quelconque des revendications précédentes, dans lequel une structure de compensation (36) d'épaisseur effective variable en relation avec sa capacité d'absorption s'étend par-dessus une pluralité de canaux de collimation (22) pour une modification du champ de rayonnement, en particulier une homogénéisation.

9. Dispositif selon l'une quelconque des revendications précédentes, dans lequel la structure de blindage (12) et la pluralité de lamelles (20) forment une structure d'une seule pièce.

10. Dispositif selon l'une quelconque des revendications précédentes, le dispositif (10) étant logé par insertion à l'intérieur d'un applicateur destiné à appliquer un rayonnement à un volume cible, en particulier sur ou à l'intérieur de l'œil.

11. Applicateur destiné à appliquer un rayonnement à un volume cible, en particulier sur ou à l'intérieur de l'œil, comprenant au moins un dispositif selon l'une quelconque des revendications 1 à 10 et au moins une source de rayonnement (14), de préférence un grain d'iode-125.

12. Dispositif selon l'une quelconque des revendications 1 à 10 ou applicateur selon la revendication 11, à utiliser en curiethérapie, en particulier en curiethérapie interstitielle ou de contact de tumeurs de la prostate, du sein, du cerveau et de l'œil, de préférence pour une curiethérapie par plaque épisclérale.

13. Procédé de fabrication d'un dispositif selon l'une quelconque des revendications 1 à 10 ou d'un applicateur selon la revendication 11, le procédé comprenant l'étape de formation du dispositif (10) ou de l'applicateur comportant le dispositif par une technique de stéréolithographie, dans lequel les lamelles (20) et éventuellement les canaux de collimation (22) sont structurés par dopage d'un matériau transmettant le rayonnement avec différentes quantités d'un matériau absorbant le rayonnement.
